# FASCICULE DE BREVET EUROPEEN

(11) **EP 2 889 049 B1**
(45) Date de publication et mention de la délivrance du brevet: **26.08.2020**
(21) Numéro de dépôt: 14200228.6
(22) Date de dépôt: 23.12.2014
(51) Int. Cl.: A61M 1/02

(54) **Réceptacle destiné à recevoir un système à poches pour le traitement du sang**
Behälter zur Aufnahme eines Beutelsystems zur Behandlung von Blut
Receptacle for receiving a system of bags for the treatment of blood

(30) Priorité: 26.12.2013 FR 1363618
(43) Date de publication de la demande: 01.07.2015
(73) Titulaire: MACO PHARMA, 59420 Mouvaux (FR)
(72) Inventeur: Deverre, Frédéric, 34090 Montpellier (FR); Chavatte, Arnaud, 62350 Saint Floris (FR); Moisan, Kevin, 59200 Tourcoing (FR)
(74) Mandataire: Sayettat, Julien Christian

(56) Documents cités:
- EP-A2- 1 736 187
- WO-A1-2004/032999
- WO-A2-2010/061866

## Description

L'invention concerne un réceptacle destiné à recevoir un système à poches pour le traitement du sang, ainsi qu'un appareil comprenant un tel réceptacle, et un procédé pour traiter un fluide biologique à l'aide d'un tel appareil.

L'invention s'applique au domaine de la transfusion sanguine, en particulier au traitement du sang, et notamment la séparation des composants sanguins.

Le sang total est constitué de deux types de composants : les cellules sanguines comprenant les globules rouges, les leucocytes et les plaquettes, et le plasma dans lequel les cellules sanguines sont en suspension. Actuellement, ne sont transfusés que les composants sanguins nécessaires aux patients.

Aujourd'hui, dans les centres de transfusion ou les hôpitaux, ces différents composants sanguins sont séparés par centrifugation : le sang total d'un donneur est recueilli dans une poche dite poche primaire d'un système à poches. Puis, le système à poches est placé dans un insert plastique, lui-même disposé dans un godet métallique d'une centrifugeuse afin de séparer les différents composants sous l'effet de la force centrifuge.

Il existe deux types de centrifugation. La centrifugation dite douce du sang total conduit à le séparer en deux couches : une couche inférieure riche en globules rouges appelée concentré de globules rouges (CGR) ; et une couche supérieure contenant le plasma, les plaquettes et les leucocytes appelée plasma riche en plaquettes (PRP). La centrifugation dite dure conduit à une séparation en trois couches : une couche inférieure de CGR ; une couche supérieure de plasma pauvre en plaquettes (PPP) ; et une couche intermédiaire formée essentiellement de leucocytes et de plaquettes, dénommée couche leuco-plaquettaire ou buffy coat.

A la fin de la centrifugation, l'opérateur retire l'insert plastique de la centrifugeuse puis retire le système à poches de l'insert plastique. Cette étape de retrait du système à poches de l'insert est critique puisqu'elle engendre un risque de mélanger à nouveau les composants sanguins qui viennent d'être séparés par centrifugation. Le système à poches est ensuite placé dans un appareil d'extraction des composants sanguin qui compresse la poche primaire, de manière à envoyer les composants sanguins séparés dans les poches satellites. Les concentrés de globules rouges ainsi extraits sont en outre à additionner d'une solution additive de type SAGM (saline - adénine - glucose - mannitol) afin de pouvoir les conserver jusqu'à 42 jours.

Un exemple d'un appareil d'extraction des composants sanguins est par exemple décrit dans le document EP 1 641 504. Cependant, la mise en place d'un système à poches sur ce type d'appareil est fastidieuse et nécessite une certaine expérience.

Il existe dans la littérature quelques appareils automatisés pour le traitement du sang tel que celui décrit dans le document EP 1 736 187. Ce type d'appareil combine les fonctions de séparation par centrifugation et d'extraction par compression. Cependant, l'ajout de la solution additive sur les CGR doit encore être réalisé manuellement.

Il existe également dans le commerce un système d'extraction dénommé Pressomat d'Hettich consistant en un insert pour centrifugeuse sous forme d'un double réceptacle, chaque réceptacle comportant une première chambre pour la poche primaire d'un système à poches, chambre dans laquelle est arrangée une poche gonflable, et une deuxième chambre pour les poches satellites du système à poches. L'insert est d'abord placé dans une centrifugeuse pour séparer les composants sanguins. Pour réaliser l'extraction des composants sanguins ainsi séparés, l'insert est ensuite placé sur un appareil d'extraction adapté, les poches satellites sont retirées de l'insert et suspendues à un crochet prévu sur l'appareil, puis la poche gonflable est gonflée afin de comprimer la poche primaire restée dans l'insert et d'extraire les composants dans les poches satellites suspendues.

Un système substantiellement équivalent est également décrit dans le document US 2011/0192745.

Ces systèmes nécessitent toujours une étape consommatrice de temps de mise en place des poches et des tubulures sur l'appareil d'extraction.

L'invention propose un réceptacle de type insert pour centrifugeuse permettant non seulement l'extraction des composants sanguins préalablement séparés mais aussi l'ajout d'une solution additive sur le CGR sans le retrait d'aucune poche du réceptacle, et ceci de façon automatisée.

A cet effet et selon un premier aspect, l'invention propose réceptacle destiné à recevoir un système à poches pour le traitement du sang comprenant une poche primaire en communication fluidique avec au moins une poche satellite, ledit réceptacle comprenant une cavité présentant un fond et une paroi latérale, ladite cavité étant divisée par une plaque de pressage en un compartiment primaire destiné à recevoir la poche primaire et en un compartiment secondaire destiné à recevoir au moins une poche satellite respectivement, ladite plaque de pressage étant agencée pour être mobile à l'intérieur de ladite cavité dans une première direction vers ladite paroi latérale de sorte à compresser la poche primaire.

Selon un deuxième aspect, l'invention concerne un appareil pour extraire au moins un composant sanguin contenu dans une poche primaire d'un système à poches, ledit système à poches comprenant en outre au moins une poche satellite de recueil dudit composant sanguin, ledit appareil comprenant :
- un réceptacle selon le premier aspect de l'invention,
- un moyen de réception dudit réceptacle, et
- un dispositif de déplacement de la plaque de pressage dans ledit réceptacle.

Selon un troisième aspect, l'invention propose un procédé pour extraire au moins un premier composant sanguin contenu dans une poche primaire d'un système à poches pour le traitement du sang, ladite poche primaire étant en communication fluidique avec au moins une poche satellite de recueil du premier composant sanguin, ledit procédé étant réalisé à l'aide d'un appareil selon le deuxième aspect de l'invention, ledit procédé comprenant les étapes de :
- disposer le système à poches dans un réceptacle selon le premier aspect de l'invention,
- disposer le réceptacle contenant le système à poches sur le moyen de réception dudit appareil,
- mettre en fonction l'appareil pour extraire ledit au moins un premier composant sanguin de la poche primaire.

D'autres objets et avantages apparaîtront au cours de la description qui suit.
Les figures 1 et 2 représentent une vue schématique d'un système à poches pour la séparation des composants sanguins comprenant une poche satellite de recueil d'un premier composant sanguin et une poche satellite de recueil du deuxième composant sanguin en dérivation et en série, respectivement.
La figure 3 représente une vue schématique d'un système à poches pour la séparation des composants sanguins comprenant une poche filtrante.
La figure 4 représente une vue schématique d'une variante de réalisation d'une poche filtrante.
La figure 5 représente une vue schématique et en éclaté d'une autre variante de réalisation d'une poche filtrante.
La figure 6 représente une vue schématique et en perspective d'un réceptacle destiné à recevoir un système à poches.
La figure 7 représente une vue schématique et en perspective du réceptacle de la figure 6 sans plaque de pressage.
La figure 8 représente une vue schématique et en perspective d'une plaque de pressage destinée à être reçue dans le réceptacle de la figure 7.
La figure 9 représente une vue schématique et en perspective du réceptacle de la figure 6 dans lequel une poche primaire, une poche filtrante et des portions de tubulures sont disposées.
La figure 10 représente de façon schématique un appareil pour l'extraction de composants sanguin.
La figure 11 représente de façon schématique un moyen de réception du réceptacle de l'appareil de la figure 10.
La figure 12 représente de façon schématique le dispositif de contrôle d'un appareil pour extraire les composants sanguins en relation avec le système à poches de la figure 3.

Lors d'un don de sang, le sang est généralement recueilli dans un système à poches comprenant une poche primaire destiné à recueillir le sang prélevé d'un donneur, et une ou plusieurs autres poches satellites pour le recueil des composants sanguins séparés par centrifugation, lesdites poches étant associées entre elles par des tubulures.

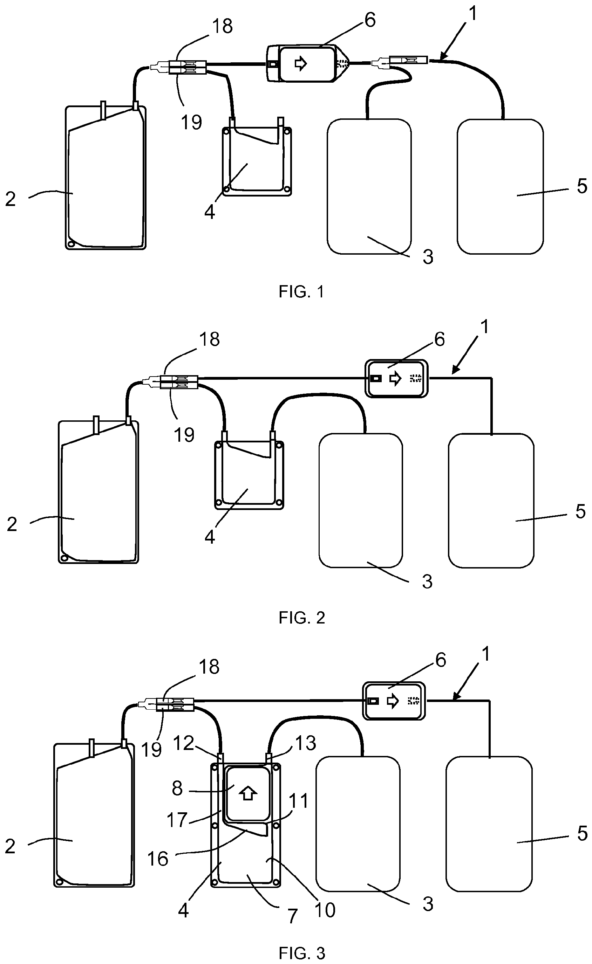

Des exemples particuliers d'un tel système à poches sont représentés sur les figures 1 à 3. Sur ces figures, le système à poches 1 comprend une poche primaire 2 destinée à contenir le sang recueilli d'un donneur.

Une fois le sang recueilli dans cette poche primaire et après centrifugation du système à poches, cette poche primaire 2 comprend au moins un premier composant sanguin et un deuxième composant sanguin. Par exemple, la poche primaire 2 contient un composant plasmatique riche en plaquettes et un composant globulaire. En variante, la poche primaire 2 contient trois composants sanguins qui sont un composant plasmatique, un composant leuco-plaquettaire et un composant globulaire.

Dans la position normale d'utilisation de la poche 2, le composant plasmatique se situe dans le haut de ladite poche, le composant globulaire dans le bas de la poche 2 et le composant leuco-plaquettaire entre le composant plasmatique et le composant plaquettaire.

Le système à poches 1 comprend en outre au moins une poche satellite 3 de recueil du premier composant sanguin en communication fluidique avec la poche primaire 2, et une poche satellite 4 de recueil du deuxième composant sanguin en communication fluidique avec la poche primaire 2.

Sur la figure 1, la poche satellite 3 de recueil du premier composant sanguin et la poche satellite 4 de recueil du deuxième composant sanguin sont disposées en parallèle ou en dérivation, c'est-à-dire que ces poches sont reliées par des tubulures différentes à la poche primaire 2 par l'intermédiaire d'un connecteur à trois branches.

Sur les figures 2 et 3, la poche satellite 4 de recueil du deuxième composant sanguin est disposée sur le chemin d'écoulement défini entre la poche primaire 2 et la poche satellite 3 de recueil du premier composant sanguin. La poche satellite 3 de recueil du premier composant sanguin et celle de recueil du deuxième composant sanguin sont donc disposées en série.

Sur les figures 1 à 3, le système à poches comprend en outre une poche satellite 5 de recueil d'un troisième composant sanguin contenu dans la poche primaire 2, ladite poche satellite 5 étant en communication fluidique avec la poche primaire 2.

Cette poche satellite 5 de recueil d'un troisième composant sanguin est notamment destinée à contenir un composant globulaire et contient avantageusement une solution additive de conservation des globules rouges, telle que par exemple une solution SAGM (saline - glucose - adénine - mannitol).

En particulier, les poches 2,3,4,5 du système à poches 1 sont reliées entre elles par des tubulures souples et sécables.

Dans une réalisation particulière représentée sur les figures 1 à 3, le système à poches 1 comprend au moins une unité de filtration 6 destinée notamment à filtrer un composant sanguin. En particulier, l'unité de filtration 6 permet d'éliminer les leucocytes du composant sanguin.

Par exemple, sur les figures 1 à 3, le système à poches comprend une unité de filtration 6 destinée à filtrer le troisième composant sanguin, tel que le composant globulaire. Cette unité de filtration est disposée sur le chemin d'écoulement défini entre la poche primaire 2 et la poche satellite 5 de recueil du troisième composant sanguin.

Sur la figure 1, l'unité de filtration 6 est disposée non seulement sur le chemin d'écoulement défini entre la poche primaire 2 et la poche satellite 5 de recueil du troisième composant sanguin mais aussi sur le chemin d'écoulement défini entre la poche primaire 2 et la poche satellite 3 de recueil du premier composant sanguin. Ainsi, l'unité de filtration 6 permet d'éliminer de façon séquentielle les leucocytes du composant plasmatique et du composant globulaire.

En variante, le système à poches 1 comprend une autre unité de filtration destinée notamment à filtrer le premier composant sanguin, tel que le composant plasmatique. Cette unité de filtration 6 est disposée sur le chemin d'écoulement défini entre la poche primaire 2 et la poche satellite 3 de recueil du premier composant sanguin.

Un exemple particulier de système à poches est représenté sur la figure 3. Dans cet exemple, la poche satellite 4 de recueil du deuxième composant sanguin inclut une unité de filtration du premier composant sanguin de sorte à former une poche filtrante 7 disposée sur le chemin d'écoulement défini entre la poche primaire 2 et la poche satellite 3 de recueil du premier composant sanguin.

En relation avec les figures 3 à 5, la poche filtrante 7 comprend un compartiment de filtration 8 disposant d'un milieu filtrant 9 et un compartiment de stockage 10 du deuxième composant sanguin, lesdits compartiments 8,10 communiquant entre eux par un passage 11 pouvant être fermé.

La poche 7 est pourvue d'un orifice d'entrée 12 et d'un orifice de sortie 13. En particulier, l'orifice d'entrée 12 est en communication avec le compartiment de stockage 10 et l'orifice de sortie 13 est en communication avec le compartiment de filtration 8.

Le milieu filtrant 9 est notamment un milieu pour éliminer les leucocytes du sang, tel que par exemple le milieu filtrant décrit dans le document EP-0 953 361 ou EP-1 336 417.

Le milieu filtrant 9 définit dans le compartiment de filtration 8 une chambre d'entrée du composant à filtrer et une chambre de sortie du composant filtré.

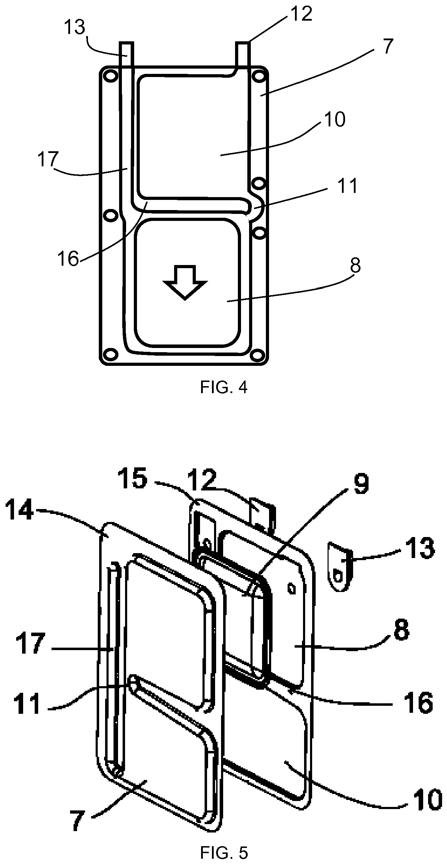

En particulier et comme montré sur la figure 5, la poche filtrante 7 comprend une première et deuxième feuille 14,15 en matière thermoplastique souple solidarisées l'une à l'autre sur leur périphérie de sorte à former un volume intérieur, ladite poche comportant un cordon de soudure 16 entre les feuilles, agencé pour former le compartiment de filtration 8 et le compartiment de stockage 10, ledit cordon de soudure 16 étant agencé pour former ledit passage 11 entre les compartiments 8,10.

Selon une réalisation, le passage 11 entre le compartiment de filtration 8 et le compartiment de stockage 10 est fermé par soudage des deux feuilles 14,15.

En relation avec les figures 3 à 5, les orifices 12,13 d'entrée et de sortie de la poche filtrante 7 sont disposés au voisinage d'un bord supérieur de la poche filtrante 7.

Cette disposition particulière des orifices d'entrée et de sortie permet d'éviter l'écrasement des tubulures reliant les différentes poches lorsque le système à poches 1 est disposé dans un insert plastique en vue de sa centrifugation ou dans un réceptacle comme décrit ci-après.

Comme illustré sur les figures 3 et 4, les orifices 12,13 d'entrée et/ou de sortie sont formés par des portions de tubulures soudées entre les feuilles 14, 15 formant la poche filtrante 7.

En variante et comme représenté sur la figure 5, les orifices 12,13 d'entrée et/ou de sortie sont formés de pièces moulées par injection, soudées sur la surface extérieure d'une ou de chacune des feuilles 14, 15 formant la poche filtrante 7.

Dans le cas où les orifices 12,13 d'entrée et/ou sortie sont disposés sur un même bord de la poche filtrante 7, le cordon de soudure 16 définissant le compartiment de filtration 8 et le compartiment de stockage 10 comprend une partie horizontale et une partie verticale qui est agencée pour former avec un bord de la poche filtrante 7, un canal 17 en communication fluidique avec l'un des orifices 12,13 d'entrée ou sortie de la poche filtrante 7.

Notamment, le compartiment de filtration 8 est disposé au dessus de la partie horizontale du cordon de soudure 16 de la poche filtrante. Ainsi, le compartiment de filtration 8 se situe au dessus du compartiment de stockage 10.

Par la suite, les termes "dessus", "dessous", "vertical", "horizontal" sont définis par rapport à la poche filtrante 7 dans sa position normale d'utilisation lors de l'extraction des composants sanguins, c'est-à-dire lorsqu'elle est placée verticalement, orifices d'entrée 12 /sortie 13 placés en haut.

Dans la configuration de la figures 5 et 3, l'orifice d'entrée 12 débouche dans le canal 17 formé par le cordon de soudure 16. Le composant sanguin qui traverse cette poche filtrante 7 s'écoule d'abord dans ce canal 17, traverse le compartiment de stockage 10, le passage 11 entre le compartiment de stockage 10 et le compartiment de filtration 8, puis traverse le compartiment de filtration 8 et ressort par l'orifice de sortie 13.

Dans la configuration de la figure 4, l'orifice de sortie 13 débouche dans le canal 17. Dans cette configuration, le composant sanguin qui traverse cette poche filtrante 7 entre d'abord par l'orifice d'entrée 12, traverse le compartiment de stockage 10, le passage 11 entre le compartiment de stockage 10 et le compartiment de filtration 8, puis traverse le compartiment de filtration 8, s'écoule dans le canal 17 et ressort par l'orifice de sortie 13.

La chambre d'entrée du compartiment de filtration 8 est en communication avec le passage 11 de la poche filtrante 7 et la chambre de sortie du compartiment de filtration 8 est en communication avec l'orifice 13 de sortie de la poche filtrante 7.

Dans une réalisation particulière non représentée, le cordon de soudure 16 comprend une partie fragile de sorte à pouvoir séparer le compartiment de filtration 8 et le compartiment de stockage 10. La séparation des compartiments 8,10 est réalisée après le recueil du deuxième composant sanguin dans le compartiment 10 dédié de la poche filtrante 7 et après fermeture du passage 11.

Par exemple, la partie fragile est constituée par une ligne de découpe comprenant des perforations.

Ce compartiment de stockage 10 séparé est ensuite stocké et/ou utilisé afin de réaliser un pool de deuxièmes composants sanguins. Le compartiment de filtration 8 peut être jeté.

Pour réaliser le pool de deuxièmes composants sanguins, la poche filtrante 7, dépourvue ou non de son compartiment de filtration 8 et dans laquelle est stockée le deuxième composant sanguin, est séparée du reste du système à poches 1. Entre quatre et sept poches filtrantes 7 sont ensuite connectées de façon stérile à un système de pool de buffy-coat pour assembler leur contenu dans une poche de transfert.

Lors de cette opération d'assemblage, le deuxième composant sanguin s'écoule dans la poche de transfert via l'orifice d'entrée 12 de chaque poche filtrante.

Dans une réalisation particulière, le cordon de soudure 16 est agencée de sorte à faciliter l'écoulement du deuxième composant sanguin en dehors du compartiment de stockage via l'orifice d'entrée 12.

En relation avec les figures 3 et 5, la partie horizontale du cordon de soudure 16 présente une épaisseur décroissante en direction de l'orifice 12 d'entrée de la poche filtrante, de sorte à créer une pente facilitant l'écoulement du deuxième composant en dehors de son compartiment de stockage 10.

Afin de contrôler l'écoulement des fluides dans le système à poches 1, certaines des tubulures et/ou des orifices d'entrée et sortie des poches comprennent un élément de fermeture comportant une zone de fragilité pouvant être rompue pour permettre l'écoulement de fluide.

Sur les figures 1 à 3, la tubulure reliant la poche primaire 2 à la poche satellite 4 de recueil du deuxième composant sanguin et la tubulure reliant la poche primaire 2 à la poche satellite 5 de recueil du troisième composant sanguin sont pourvues chacune d'un tel élément de fermeture 18,19.

Pour extraire au moins un premier composant sanguin contenu dans la poche primaire 2 d'un système à poches 1 tel que décrit ci-dessus en relation avec les figures 1 à 3, on compresse la poche primaire 2 de sorte à le transférer dans la poche satellite 3 de recueil du composant.

Quand la poche primaire contient un deuxième et un troisième composant sanguin, en poursuivant la compression de la poche primaire 2, il est possible de transférer le deuxième composant sanguin dans une poche satellite 4 de recueil du deuxième composant sanguin et le troisième composant sanguin dans une poche satellite 5 de recueil du troisième composant sanguin.

Un premier exemple particulier de procédé pour extraire au moins un premier et deuxième composant contenus dans la poche primaire 2 d'un système à poches comme illustré sur la figure 2 est maintenant décrit ci-après.

Dans ce procédé, le système à poches comprend en outre au moins une poche satellite 3 de recueil du premier composant sanguin en communication fluidique avec la poche primaire 2, et une poche satellite 4 de recueil du deuxième composant sanguin disposée sur le chemin d'écoulement défini entre la poche primaire 2 et la poche satellite 3 de recueil du premier composant sanguin.

Le premier composant sanguin est par exemple un composant plasmatique et le deuxième composant sanguin, un composant leuco-plaquettaire.

Les poches satellites 3,4 de recueil du premier et deuxième composant sanguin sont ainsi disposées en série.

Ledit procédé de cet exemple comprend les étapes de :
- contraindre la poche satellite 4 de recueil du deuxième composant sanguin selon un premier volume,
- compresser la poche primaire 2 de sorte à extraire le premier composant sanguin de la poche primaire 2 et le transférer au moins en partie dans la poche satellite 3 de recueil du premier composant sanguin en passant par la poche satellite 4 de recueil du deuxième composant sanguin contrainte selon le premier volume,
- après l'extraction du premier composant sanguin de la poche primaire 2, contraindre la poche satellite 4 de recueil du deuxième composant sanguin selon un deuxième volume,
- poursuivre la compression de la poche primaire 2 de sorte à extraire le deuxième composant sanguin de la poche primaire 2 et le transférer au moins en partie dans la poche satellite 4 de recueil du deuxième composant sanguin contrainte selon le deuxième volume.

La contrainte appliquée sur la poche satellite 4 de recueil du deuxième composant sanguin évite le gonflement de cette poche lorsque celle-ci reçoit le premier composant sanguin et permet donc un transfert plus rapide du premier composant sanguin dans la poche satellite 3 dédiée.

Le premier volume de contrainte de la poche satellite 4 de recueil du deuxième composant sanguin est ainsi choisi de sorte à permettre un écoulement rapide du composant sanguin au travers de cette poche satellite 4. Il est donc minimal, par exemple inférieur à 20 ml.

Encore plus avantageusement, le premier volume de contrainte est déterminé en fonction du volume de premier composant sanguin que l'on souhaite obtenir dans le volume de deuxième composant sanguin.

En particulier, dans le cas d'un composant leuco-plaquettaire, il est recommandé d'avoir un volume de composant plasmatique d'environ 20 à 40 ml dans le composant leuco-plaquettaire final afin de mettre les plaquettes en suspension. Ainsi, le premier volume de contrainte de la poche satellite 4 de recueil du deuxième composant sanguin est compris entre 20 et 40 ml, particulièrement entre 25 et 35 ml.

Après le transfert d'au moins une partie du premier composant sanguin dans la poche satellite 3 dédiée, la poche satellite 4 de recueil du deuxième composant sanguin est contrainte selon un deuxième volume. Notamment, le deuxième volume est supérieur au premier volume.

Ce deuxième volume correspond au volume de deuxième composant sanguin, notamment le volume final de composant leuco-plaquettaire, que l'on souhaite obtenir. Par exemple, le deuxième volume de contrainte est compris entre 40 et 80 ml, notamment entre 50 et 60 ml.

Ce procédé permet donc de maîtriser à la fois le volume final de composant leuco-plaquettaire et le volume de composant plasmatique diluant le composant leuco-plaquettaire.

Dans un mode de réalisation particulier, après transfert d'au moins une partie du premier composant sanguin dans la poche satellite 3 de recueil du premier composant sanguin, le procédé comprend l'étape de couper la communication fluidique entre la poche satellite 3 de recueil du premier composant sanguin et la poche satellite 4 de recueil du deuxième composant sanguin contrainte selon le premier volume.

Le transfert du premier composant sanguin est suivi à l'aide d'un détecteur optique placé au niveau de la poche primaire 2 ou de la poche satellite 4 de recueil du deuxième composant sanguin ou encore de la tubulure entre la poche primaire 2 et ladite poche satellite 4.

Lorsque l'interface entre le premier composant sanguin et le deuxième composant sanguin est détecté par ce détecteur, on ferme la communication fluidique entre la poche satellite 3 de recueil du premier composant sanguin et la poche satellite 4 de recueil du deuxième composant sanguin.

Lorsque le système à poches 1 comprend une poche satellite 5 de recueil d'un troisième composant sanguin contenu dans la poche primaire 2, ladite poche satellite étant en communication fluidique avec la poche primaire, ledit procédé comprend en outre l'étape de :
- après l'extraction du deuxième composant sanguin de la poche primaire 2, poursuivre la compression de la poche primaire de sorte à extraire le troisième composant sanguin de la poche primaire 2 et le transférer dans la poche satellite 5 de recueil du troisième composant sanguin.

Le troisième composant sanguin est par exemple un composant globulaire.

Dans le cas d'un système à poches comprenant une poche filtrante 7, il est également envisagé d'appliquer une contrainte selon un premier et un deuxième volume sur la poche filtrante 7 et notamment le compartiment de stockage 10 du deuxième composant sanguin de la poche filtrante 7.

Le procédé selon ce deuxième exemple comprend alors les étapes du procédé selon le premier exemple dans lequel la poche satellite 4 de recueil du deuxième composant sanguin est remplacée par une poche filtrante 7.

Dans ce cas, la poursuite de la compression de la poche primaire 2 est réalisée de sorte à transférer le deuxième composant sanguin dans le compartiment de stockage 10 de la poche filtrante 7.

Le procédé selon ce deuxième exemple comprend en outre l'étape de fermer le passage 11 entre le compartiment de stockage 10 et le compartiment de filtration 8.

Un procédé selon un troisième exemple est décrit ci-dessous, utilisant également une poche filtrante 7.

Ce procédé est adapté pour extraire au moins un premier et un deuxième composant sanguin contenus dans une poche primaire 2 d'un système à poches 1, ledit système à poches comprenant en outre au moins une poche satellite 3 de recueil du premier composant sanguin en communication fluidique avec la poche primaire 2, et une poche filtrante 7 disposée sur le chemin d'écoulement défini entre la poche primaire 2 et la poche satellite 3 de recueil du premier composant sanguin.

Comme déjà décrit, cette poche filtrante 7 comprend un compartiment de filtration 8 disposant d'un milieu filtrant et un compartiment de stockage 10 du deuxième composant sanguin, lesdits compartiments 8,10 communiquant entre eux par un passage 11 pouvant être fermé.

Le procédé selon le troisième exemple comprend les étapes de :
- compresser la poche primaire 2 de sorte à extraire le premier composant sanguin de la poche primaire 2 et à le transférer au moins en partie dans la poche satellite 3 de recueil du premier composant sanguin en passant par ladite poche filtrante 7,
- poursuivre la compression de la poche primaire 2 de sorte à extraire le deuxième composant sanguin de la poche primaire 2 et à le transférer au moins en partie dans le compartiment de stockage 10 de ladite poche filtrante 7, et
- fermer le passage 11 entre le compartiment de stockage 10 et le compartiment de filtration 8.

Avantageusement, le passage 11 entre les deux compartiments est fermé lorsque le compartiment de stockage 10 comprend une quantité prédéterminée de premier composant. Cette quantité peut être nulle, c'est-à-dire que le premier composant est transféré en totalité dans la poche satellite 3 de recueil du premier composant sanguin.

En particulier, seul le premier composant sanguin est filtré. Ainsi, lors de la compression de la poche primaire 2, le premier composant sanguin est transféré au moins en partie dans la poche satellite 3 de recueil du premier composant sanguin en passant d'abord dans le compartiment de stockage 10 de la poche filtrante 7 puis dans le compartiment de filtration 8 de ladite poche filtrante.

Dans ce troisième exemple, il est également envisagé les étapes suivantes :
- avant la compression de la poche primaire 2, contraindre le compartiment de stockage 10 de la poche filtrante 7 selon un premier volume, et
- après l'extraction du premier composant sanguin de la poche primaire, contraindre le compartiment de stockage 10 de la poche 7 filtrante selon un deuxième volume.

Avantageusement, une contrainte est également appliquée sur le compartiment de filtration 8 de sorte à filtrer sous pression le premier composant sanguin en évitant le gonflement du compartiment de filtration 8. Cette filtration sous pression réduit la perte de composant sanguin dans le compartiment de filtration 8 et améliore la qualité de la filtration.

Ainsi, le procédé selon le troisième exemple comprend avantageusement l'étape de :
- avant la compression de la poche primaire 2, contraindre le compartiment de filtration 10 de la poche filtrante selon un troisième volume.

Le troisième volume est minimal de sorte à minimiser la perte résiduelle en premier composant résultant de la filtration.

Un quatrième procédé est décrit utilisant le système à poches 1 de la figure 2 dans lequel la poche satellite 3 de recueil du premier composant sanguin et la poche satellite 4 de recueil du deuxième composant sanguin sont disposées en série. Le système comprend également en parallèle une poche satellite 5 de recueil du troisième composant sanguin.

Le procédé est notamment adapté pour extraire d'une poche primaire 2 un premier composant sanguin tel qu'un composant plasmatique riche en plasma et un deuxième composant sanguin tel qu'un composant globulaire. Le procédé comprend les étapes de :
- contraindre la poche satellite 4 de recueil du deuxième composant sanguin selon un premier volume,
- compresser la poche primaire 2 de sorte à extraire le premier composant sanguin de la poche primaire 2 et le transférer au moins en partie dans la poche satellite 3 de recueil du premier composant sanguin en passant par la poche satellite 4 de recueil du deuxième composant sanguin contrainte selon le premier volume,
- poursuivre la compression de la poche primaire 2 de sorte à extraire le deuxième composant sanguin de la poche primaire et le transférer au moins en partie dans la poche satellite 5 de recueil du troisième composant sanguin.

Dans ce cas, aucun composant sanguin n'est stocké dans la poche satellite 4 de recueil du deuxième composant sanguin, le deuxième composant sanguin étant transféré dans la poche satellite 5 de recueil du troisième composant sanguin.

Ainsi, un même système à poches tel que celui représenté sur la figure 2 est utilisé au choix de l'utilisateur pour extraire deux ou trois composants sanguins contenus dans la poche primaire. Plus particulièrement, l'utilisateur peut décider au moment de la séparation de récupérer un composant leuco-plaquettaire ou non, ce qui offre une grande flexibilité par rapport aux systèmes à poches actuel dans lequel ce choix doit être fait au moment du don.

Pour mettre en œuvre ces procédés d'extraction, il est prévu un appareil pour extraire au moins un composant sanguin contenu dans une poche primaire 2.

Un appareil pour mettre en œuvre au moins l'un des procédés décrits ci-dessus comprend au moins un moyen de compression de la poche primaire 2 de sorte à extraire au moins un composant sanguin, particulièrement un premier et un deuxième composant sanguin et encore plus particulièrement, un premier, deuxième et troisième composant sanguin.

Selon une réalisation, le moyen de compression comprend d'une part une paroi de pressage et d'autre part une plaque de pressage entre lesquelles la poche primaire 2 est destinée à être placée. La plaque de pressage est mobile en translation pour venir comprimer la poche primaire 2.

La plaque de pressage est notamment un élément peu épais ayant une surface substantiellement plane. La plaque de pressage est rigide de sorte à pouvoir compresser la poche primaire 2, notamment contre la paroi de pressage, sans se déformer.

Le moyen de compression comprend en outre un dispositif de déplacement de ladite plaque de pressage.

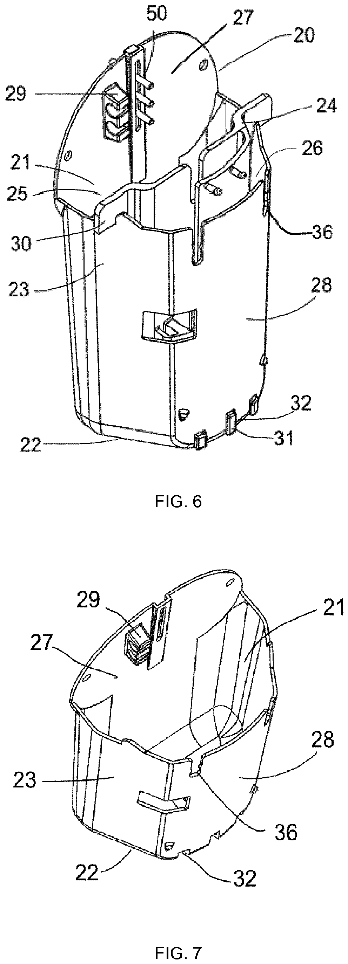

Selon une réalisation représentée sur les figures 6 à 11 et selon l'invention, la paroi de pressage et la plaque de pressage font partie d'un réceptacle 20 destiné à recevoir un système à poches 1.

Plus en détail et en relation avec les figures 6 à 11, l'appareil selon l'invention comprend un réceptacle 20 destiné à recevoir un système à poches 1 comprenant une poche primaire 2 en communication fluidique avec au moins une poche satellite 3,5. Ce réceptacle 20 est en particulier adapté pour être disposé dans un godet d'une centrifugeuse.

Comme illustré sur la figure 6, le réceptacle 20 comprend une cavité 21 présentant un fond 22 et une paroi latérale 23, ladite cavité 21 étant divisée par une plaque de pressage 24 en un compartiment primaire 25 destiné à recevoir la poche primaire 2 et en un compartiment secondaire 26 destiné à recevoir au moins une poche satellite 3,5 respectivement.

Sur les figures, la cavité 21 présente substantiellement une forme en demi-cylindre creux dont la base correspond au fond 22 de la cavité.

Par la suite, la partie plane de la surface latérale du demi-cylindre constitue la paroi latérale arrière 27 de la cavité 21 et la partie arrondie, la paroi latérale avant 28.

La paroi latérale arrière 27 s'étend au-delà du demi-cylindre et comprend avantageusement des logements 29 pour tubulures, comme expliqué ci-après.

La plaque de pressage 24 est notamment substantiellement perpendiculaire au fond 22 de la cavité de sorte que la poche primaire 2 et les poches satellites 3,5 sont disposées à la verticale dans le réceptacle 20.

Comme illustré sur la figure 6, le compartiment primaire 25 du réceptacle se situe à l'arrière de la cavité, à l'endroit le plus large du demi-cylindre, et le compartiment secondaire 26 à l'avant.

La paroi latérale arrière 27 de la cavité 21 constitue ainsi la paroi fixe de pressage de l'appareil contre laquelle la poche primaire 2 est compressée.

La plaque de pressage 24 est agencée pour être mobile à l'intérieur de la cavité 21 dans une première direction vers ladite paroi latérale 23 de sorte à pouvoir compresser la poche primaire 2.

La plaque de pressage 24 et la paroi latérale de la cavité 21 forment ensemble un moyen de compression de la poche primaire 2.

Lorsque la plaque de pressage 24 compresse la poche primaire 2 contre la paroi latérale 23 de la cavité, au moins une partie du contenu de la poche primaire 2 est extraite de ladite poche primaire 2 et transférée vers une poche satellite 3,4,5. Par exemple, en compressant la poche primaire 2, il est possible d'extraire un premier composant sanguin tel qu'un composant plasmatique vers une poche satellite 3 de recueil du premier composant.

En continuant à compresser la poche primaire 2, il est également possible d'extraire un deuxième composant sanguin de la poche primaire 2 et de le transférer dans une poche satellite 4 de recueil du deuxième composant sanguin.

Dans le cas où la poche primaire 2 contient un troisième composant, celui-ci peut être extrait par compression de la poche primaire 2 et transféré vers une poche satellite 5 de recueil du troisième composant sanguin.

Selon une réalisation particulière, la plaque de pressage 24 est mobile à l'intérieur de la cavité dans une deuxième direction, opposée à la première direction, vers la paroi latérale 28 de sorte à pouvoir compresser ladite au moins poche satellite 3,5.

Le déplacement de la plaque de pressage 24 dans la deuxième direction permet par exemple de chasser l'air contenu dans une poche satellite 3 contenant un composant sanguin tel qu'un composant plasmatique et/ou de transférer une solution additive contenue dans une poche satellite 5 vers la poche primaire 2 contenant un composant sanguin séparé tel qu'un composant globulaire.

Pour pouvoir déplacer la plaque de pressage 24, celle-ci est pourvue de saillies dépassant du réceptacle 20 et sur lesquelles une pression est exercée afin de déplacer la plaque de pressage 24 à l'intérieur de la cavité du réceptacle 20 dans un sens ou dans l'autre.

Sur les figures, les saillies sont sous forme d'ailettes 30 et d'ergots 31. La plaque de pressage 24 comporte une paire d'ailettes 30 disposée sur le haut de la plaque de pressage 24 et trois ergots 31 disposés sur le bas de la plaque de pressage 24.

Les ergots 31 sont agencés pour s'engager dans des ouvertures 32 pratiquées dans la paroi latérale 23 de la cavité 21.

En variante, la plaque de pressage 24 est amovible, c'est-à-dire qu'elle peut être retirée du réceptacle. Dans ce cas, la mise en place du système à poches dans la cavité est simplifiée. Il suffit de positionner la poche primaire 2 et/ou les poches satellites 3,4,5 de part et d'autre de ladite plaque de pressage 24 puis de glisser l'ensemble de la plaque de pressage 24 et des poches 2,3,4,5 dans la cavité 21 du réceptacle 20.

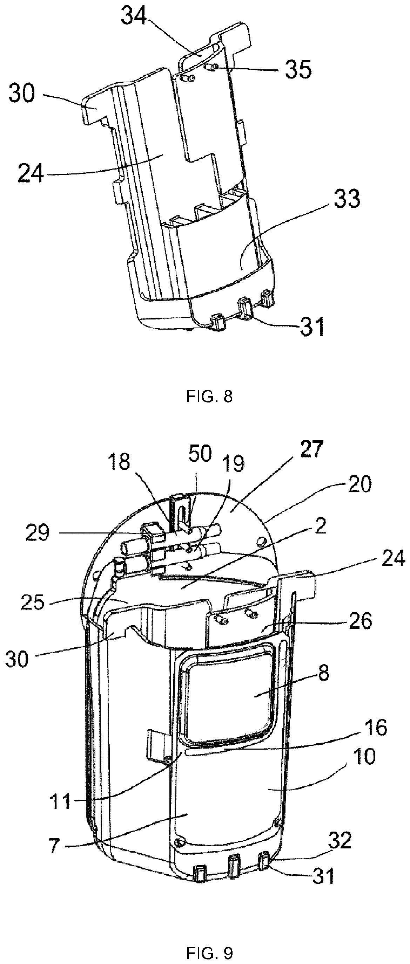

En relation avec la figure 8, pour faciliter le positionnement des poches satellites 3,4,5, la plaque de pressage 24 comprend une bordure formant une rainure 33 sur un ou plusieurs bords de la plaque de pressage 24, dans lesquelles la ou les poches satellites 3,4,5 peuvent être glissées.

Avantageusement, la géométrie de la surface de la plaque de pressage 24 en contact avec la poche primaire 2 correspond sensiblement à la géométrie de la poche primaire 2 remplie. Ainsi, la pression exercée sur la poche primaire 2 lors du déplacement de la plaque de pressage 24 est substantiellement constante sur toute la surface de la poche primaire 2, ce qui permet une meilleure extraction des composants sanguins.

Sur la figure 8, la surface arrière de la plaque de pressage 24 est substantiellement concave.

La géométrie de la surface de la plaque de pressage 24 en contact avec ladite au moins une poche satellite 3,5 correspond sensiblement à la géométrie de la paroi latérale de la cavité 21 vers laquelle la plaque de pressage 24 est déplacée pour comprimer ladite poche satellite 3,5. Cette correspondance de géométrie permet notamment de vidanger complètement la poche satellite 5 contenant une solution additive.

Sur la figure 8, la surface avant de la plaque de pressage 24 présente substantiellement une surface arrondie dont la géométrie correspond à la géométrie de la surface intérieure de paroi latérale 23 avant de la cavité 21.

Lorsque le système à poches 1 comprend une unité de filtration 6, il est avantageux de prévoir que le réceptacle 20 et notamment la plaque de pressage 24 comprenne un logement 34 pour une telle unité de filtration 6.

Ainsi, l'unité de filtration 6 reste en place durant la centrifugation, ce qui permet d'assurer son intégrité et/ou l'intégrité des poches du système à poches 1.

En relation avec la figure 8, le logement 34 pour l'unité de filtration 6 est arrangé sur la plaque de pressage 24 et comprend avantageusement une ouverture en haut et en bas du logement afin de faciliter le passage des tubulures.

Aussi, afin de maintenir les poches en position verticale dans le réceptacle 20, il est utile de pourvoir ledit réceptacle 20 d'une ou plusieurs tiges 35 destinées à recevoir des œillets pourvus sur la poche primaire 2 et/ou satellites 3,4,5.

Sur la figure 8, deux tiges 35 sont arrangées sur la surface avant de la plaque de pressage 24 et notamment sur la surface avant du logement 34 pour l'unité de filtration 6. Ces tiges 35 maintiennent en position verticale au moins une poche satellite 3,5.

Selon une réalisation particulière, la paroi latérale 23 de la cavité 21 est pourvue sur sa surface extérieure d'un moyen d'association d'une poche satellite, notamment de la poche satellite 4 de recueil d'un deuxième composant sanguin.

Par exemple, ce moyen d'association est une paire d'ouvertures 36 pratiquée dans la paroi latérale avant 28 de la cavité 21, dans laquelle les orifices 12,13 d'entrée et sortie d'une poche satellite 4,7 se glissent. Pour cela, chacun des orifices 12,13 d'entrée et sortie de ladite poche satellite se présente sous forme d'une pièce injectée soudée sur la surface latérale de ladite poche.

La configuration particulière de ce réceptacle 20 avec notamment sa plaque de pressage mobile 24 permet le transfert des composants sanguins séparés sans retirer aucune poche du réceptacle 20 ce qui est avantageux pour au moins deux raisons.

D'abord, le système à poches 1 dans son ensemble, et plus particulièrement la poche primaire 2, n'est pas retiré du réceptacle 20, ce qui limite le risque de mélanger à nouveau les composants sanguins séparés par centrifugation. Ensuite, aucune manipulation du système à poches 1 n'est nécessaire, ce qui est un gain de temps pour l'opérateur qui n'a qu'à positionner le réceptacle 20 sur un appareil dédié afin de réaliser l'extraction des composants dans les différentes poches satellites.

Dans une réalisation non représentée, le réceptacle 20 est adossé ou intégré à un autre réceptacle 20, de sorte à former un réceptacle jumelé comprenant deux cavités 21 destinées à recevoir chacune un système à poches 1.

De façon avantageuse, le réceptacle 20, dans sa version simple ou jumelée, est adapté pour être disposé dans un godet d'une centrifugeuse. La place disponible dans le réceptacle 20 pour recevoir le système à poche est optimisée grâce à la plaque de pressage mobile 24 qui non seulement divise la cavité 21 du réceptacle 20 en deux compartiments 25,26 mais aussi permet la compression des poches du système à poches.

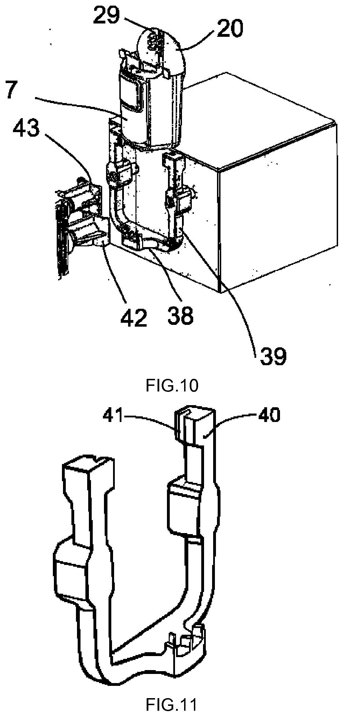

En relation avec les figures 10 et 11 et selon un exemple particulier de l'invention, un appareil est décrit pour extraire au moins un composant sanguin contenu dans une poche primaire 2 d'un système à poches 1, ledit système à poches 1 comprenant en outre au moins une poche satellite 3,5 de recueil dudit composant sanguin.

L'appareil comprend :
- un réceptacle 20 tel que décrit ci-dessus,
- un moyen de réception 38 dudit réceptacle 20, et
- un dispositif de déplacement 39 de la plaque de pressage 24 dans ledit réceptacle 20.

Le dispositif de déplacement 39 de la plaque de pressage 24 comprend notamment un moyen d'engagement de la plaque de pressage et un actionneur en translation dudit moyen d'engagement présentant par exemple un moteur en liaison avec une vis sans fin.

Le moyen d'engagement comprend un support 40 muni de fentes 41 dans lesquelles les saillies de la plaque de pressage 24 viennent s'engager.

Le dispositif de déplacement 39 de la plaque de pressage 24 est agencé pour permettre le déplacement de la plaque de pressage 24 à l'intérieur de la cavité 21 du réceptacle 20 dans une première direction. Avantageusement, le déplacement de la plaque de pressage 24 est possible dans une première direction et dans une deuxième direction opposée à la première direction.

Comme déjà décrit ci-dessus, dans le cas d'un système à poches 1 tel que représenté en figure 2 et 3, dans lequel la poche satellite 3 de recueil d'un premier composant sanguin et la poche satellite 4 de recueil d'un deuxième composant sanguin ou la poche filtrante 7, sont disposées en série, il est envisagé de disposer ladite poche de recueil 4 du deuxième composant sanguin ou la poche filtrante 7 sur la surface extérieure de la paroi latérale 23 de la cavité 21 du réceptacle 20, comme représenté sur la figure 9.

Dans ce cas, pour permettre de transférer le premier composant sanguin dans la poche satellite 3 de recueil du premier composant sanguin en passant par la poche satellite 4 de recueil du deuxième composant sanguin ou la poche filtrante 7, un procédé particulier consiste à contraindre la poche satellite 4 de recueil du deuxième composant sanguin ou le compartiment de stockage 10 de la poche filtrante 7 selon un premier volume, suffisamment faible pour permettre un transfert rapide et complet du premier composant sanguin dans sa poche satellite 3 dédiée.

Aussi, après l'extraction du premier composant sanguin de la poche primaire 2, le procédé consiste à contraindre la poche satellite 4 de recueil du deuxième composant sanguin ou le compartiment de stockage 10 de la poche filtrante 7 selon un deuxième volume, qui correspond généralement au volume désiré de deuxième composant sanguin.

Pour cela, l'appareil comprend en outre un moyen de contrainte de la poche de recueil 4 du deuxième composant sanguin ou du compartiment de stockage 10 de la poche filtrante 7 selon un premier et un deuxième volume.

Le moyen de contrainte comprend une paroi fixe de contrainte et une plaque de contrainte entre lesquelles la poche satellite 4 de recueil du deuxième composant sanguin est destinée à être placée. La plaque de contrainte est mobile en translation pour venir contraindre ladite poche satellite.

Le moyen de contrainte comprend en outre un dispositif de déplacement de la plaque de contrainte.

Afin de fixer ou déterminer le premier et le deuxième volume de contrainte, il est avantageux de prévoir en outre un capteur de pression sur la plaque de contrainte.

En particulier, lorsque l'appareil comprend un réceptacle 20 tel que décrit ci-dessus, la paroi fixe de contrainte correspond à la paroi latérale avant 28 de la cavité 21 du réceptacle 20.

L'appareil comprend alors une plaque de contrainte 42 mobile pour contraindre une poche satellite disposée sur la surface extérieure du réceptacle 20 et un dispositif de déplacement de ladite plaque de contrainte. En particulier, la plaque de contrainte 42 est agencée pour contraindre une poche satellite, notamment une poche satellite 4 de recueil du deuxième composant sanguin selon un premier et un deuxième volume.

Le dispositif de déplacement de la plaque de contrainte 42 comprend notamment un moyen d'engagement de la plaque de contrainte et un actionneur en translation dudit moyen d'engagement présentant par exemple un moteur en liaison avec une vis sans fin.

La détermination ou la fixation du volume de composant sanguin dans la poche de recueil 4 ou le compartiment de stockage 10 du deuxième composant sanguin dépend non seulement de la distance entre la paroi de contrainte et la plaque de contrainte, mais aussi de la pression détectée sur ladite plaque de contrainte.

Dans une autre réalisation, dans le cas où le système à poches 1 destiné à être disposé dans un réceptacle 20 comprend une poche filtrante 7 comme décrit ci-dessus et illustré sur la figure 3, la plaque de contrainte 42 est agencée pour contraindre le compartiment de stockage 10 du deuxième composant sanguin de la poche filtrante 7.

En autre variante représentée sur la figure 10, une autre plaque de contrainte 43 est agencée pour contraindre le compartiment de filtration 8 de la poche filtrante 7 pour réaliser une filtration sous pression.

En variante non représentée, la plaque de contrainte 42 est agencée pour contraindre non seulement le compartiment de stockage 10 de la poche filtrante 7 mais aussi le compartiment de filtration 8.

L'appareil comprend en outre un dispositif de contrôle destiné à contrôler l'écoulement des fluides dans le système à poches 1.

En particulier, le dispositif de contrôle comprend un ensemble de clamps pour les tubulures associant entre elles la poche primaire 2 et les poches satellites 3,4,5.

Dans le cas où le système à poches 1 destiné à être disposé dans le réceptacle 20 comprend une poche filtrante 7 comme décrit ci-dessus et illustré sur la figure 3, le dispositif de contrôle comprend en outre au moins un clamp 46 pour fermer le passage 11 entre le compartiment de stockage 10 et le compartiment de filtration 8 de la poche filtrante 7.

Par exemple, le dispositif de contrôle comprend entre un et six clamps. Chaque clamp est apte à pincer une tubulure du système à poches 1 pour bloquer l'écoulement fluidique à l'intérieur de ladite tubulure.

Dans un exemple particulier, les clamps comprennent une soudeuse apte à souder la tubulure de sorte à fermer définitivement l'écoulement dans ladite tubulure, après extraction et transfert des composants sanguins séparés dans les poches appropriées.

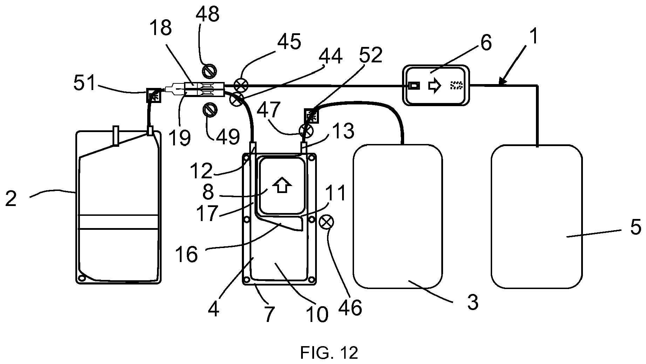

Selon la figure 12, lorsqu'on dispose le système à poches dans l'appareil, la tubulure reliant la poche primaire 2 et la poche filtrante 7 est positionnée dans un premier clamp 44 ; la tubulure reliant la poche primaire 2 et la poche satellite 5 de recueil du troisième composant sanguin est positionnée dans un deuxième clamp 45 ; le passage 11 entre le compartiment de stockage 10 et le compartiment de filtration 8 de la poche filtrante 7 est positionné dans un troisième clamp 46 ; la tubulure reliant la poche filtrante 7 et la poche satellite 3 de recueil du premier composant sanguin est positionnée dans un quatrième clamp 47.

Chaque clamp est en outre muni d'un détecteur optique afin de signaler le bon positionnement de la tubulure dans le clamp.

Selon une réalisation, le dispositif de contrôle comprend un ensemble de dispositifs de rupture 48,49 d'au moins un élément de fermeture 18,19 comportant une zone de fragilité, ledit élément de fermeture étant disposé à l'intérieur de l'une des tubulures associant entre elles la poche primaire 2 et une poche satellite 3,4,5. Un tel dispositif de rupture est décrit dans le document FR 2 968 568.

En particulier, chacun desdits dispositifs de rupture comporte un ensemble de réception d'au moins une tubulure, ledit ensemble comprenant un élément fixe pourvu d'un premier logement 29 destiné à recevoir une première portion de ladite tubulure, un élément mobile 50 pourvu d'un deuxième logement destiné à recevoir une deuxième portion de ladite tubulure souple, et un organe d'entraînement en déplacement de l'élément mobile, de sorte à pouvoir provoquer la rupture de la zone de fragilité de l'élément de fermeture lorsque la tubulure est placée dans l'ensemble de réception.

Sur la figure 6, le logement 29 de l'élément fixe du dispositif de rupture est arrangé sur la paroi latérale arrière 27 de la cavité 21 du réceptacle 20. L'élément mobile 50 est sous forme de tiges qui traversent une ouverture pratiquée dans la paroi latérale de la cavité pour venir recevoir la deuxième portion de la tubulure.

L'appareil comprend en outre un dispositif de détection afin de détecter l'interface entre les différents composants sanguins et/ou le passage d'un composant dans les tubulures du système à poches 1.

En particulier, le dispositif de détection comprend un ensemble de détecteurs optiques placés au niveau de la poche primaire 2 et/ou des tubulures du système à poches 1 afin de détecter l'interface entre les différents composants sanguins et/ou le passage d'un composant dans les tubulures.

Les détecteurs optiques sont arrangés sur le réceptacle 20 ou directement sur l'appareil.

Selon la figure 12, une fois le système à poches disposé dans l'appareil, un premier détecteur 51 est positionné à proximité de la sortie de la poche primaire 2, sur la tubulure reliant la poche primaire 2 aux poches satellite 3,4,5. Ce détecteur détecte l'interface entre les différents composants sanguins contenus dans la poche primaire 2, mais aussi l'interface entre de l'air et un composant sanguin.

Un deuxième détecteur 52 est positionné à la sortie de la poche satellite 4 de recueil du deuxième composant sanguin ou de la poche filtrante 7 et permet de détecter l'interface entre l'air et le composant plasmatique.

Dans une réalisation particulière, le dispositif de détection comprend un ou plusieurs détecteurs de pression agencés sur la plaque de contrainte afin de déterminer la pression exercée par la poche satellite de recueil du deuxième composant sanguin. L'atteinte d'une pression déterminée traduit l'atteinte du volume attendu de deuxième composant sanguin.

L'appareil comprend également un système de pilotage apte à commander les dispositifs de déplacement et de contrôle, de sorte à transférer les composants sanguins de la poche primaire 2 dans les poches satellites 3,4,5.

Le système de pilotage comprend notamment un microprocesseur conçu pour exécuter un programme de commandes. L'exécution de ce programme permet au système de pilotage de piloter les dispositifs de déplacement et de contrôle en fonction par exemple des signaux reçus par les détecteurs optiques et/ou les détecteurs de pression.

Le système de pilotage permet de mettre en œuvre un procédé de traitement d'un fluide biologique comme décrit ci-dessus.

Enfin, il est décrit un procédé selon l'invention pour extraire au moins un premier composant sanguin contenu dans une poche primaire 2 d'un système à poches 1, ladite poche primaire étant en communication fluidique avec au moins une poche satellite 4 de recueil du premier composant sanguin, ledit procédé étant réalisé à l'aide d'un appareil tel que décrit ci-dessus. Le procédé comprend les étapes de :
- disposer un système à poches 1 comprenant une poche primaire 2 contenant au moins un premier composant sanguin séparé dans le réceptacle 20 de l'appareil,
- disposer ledit réceptacle 20 contenant le système à poches 1 sur le moyen de réception 38 dudit appareil,
- mettre en fonction l'appareil pour extraire ledit au moins un composant sanguin de la poche primaire 2.

Ainsi, il est possible de réaliser l'extraction des différents composants sanguins d'un système à poches 1, sans manipulation de celui-ci une fois placé dans le réceptacle 20 au début du processus. Aucune manipulation du système à poches 1 n'est nécessaire, ce qui est un gain de temps pour l'opérateur qui n'a qu'à positionner le réceptacle 20 sur un appareil dédié afin de réaliser l'extraction des composants dans les différentes poches satellites 3,4,5.

Notamment, la mise en fonction de l'appareil comprend, via le système de pilotage, l'action de déplacer la plaque de pressage 24 du réceptacle 20 pour compresser la poche primaire 2 du système à poches 1 de sorte à extraire le premier composant de la poche primaire 2 et à le transférer dans la poche satellite 3.

Dans le cas d'un système à poches 1 comprenant une poche satellite de recueil 4 d'un deuxième composant sanguin disposée en série (figure 2), et dans le cas d'un appareil comprenant une plaque de contrainte mobile 42 pour contraindre cette poche satellite disposée sur la surface extérieure du réceptacle, la mise en fonction de l'appareil comprend les actions de :
- déplacer la plaque de contrainte 42 pour contraindre la poche satellite 4 de recueil du deuxième composant sanguin selon un premier volume,
- déplacer la plaque de pressage 24 pour compresser la poche primaire 2 de sorte à extraire le premier composant sanguin de la poche primaire 2 et le transférer au moins en partie dans la poche satellite 3 de recueil du premier composant sanguin en passant par la poche satellite 4 de recueil du deuxième composant sanguin contrainte selon le premier volume,
- après l'extraction du premier composant sanguin, déplacer la plaque de contrainte 42 pour contraindre la poche satellite 4 de recueil du deuxième composant sanguin selon un deuxième volume,
- déplacer la plaque de pressage 24 pour poursuivre la compression de la poche primaire 2 de sorte à extraire le deuxième composant sanguin de la poche primaire et le transférer au moins en partie dans la poche satellite 4 de recueil du deuxième composant sanguin contrainte selon le deuxième volume.

En relation avec un système à poches 1 comprenant une poche filtrante 7, la mise en fonction de l'appareil comprend les actions de :
- déplacer la plaque de pressage 24 pour compresser la poche primaire 2 de sorte à extraire le premier composant sanguin de la poche primaire 2 et à le transférer au moins en partie dans la poche satellite 3 de recueil du premier composant sanguin en passant par ladite poche filtrante 7,
- poursuivre le déplacement de la plaque de pressage 24 pour poursuivre la compression de la poche primaire 2 de sorte à extraire le deuxième composant sanguin de la poche primaire 2 et à le transférer au moins en partie dans le compartiment de stockage 10 de ladite poche filtrante 7, et
- actionner le dispositif de contrôle pour fermer le passage 11 entre le compartiment de stockage 10 et le compartiment de filtration 8.

Selon un exemple particulier, un système à poches 1 tel que celui de la figure 3 est disposé dans un réceptacle 20 comme montré partiellement sur la figure 9. En relation avec la figure 12, le système de pilotage exécute les actions suivantes :
- fermer les clamps 44,45 à la sortie de la poche primaire 2,
- rompre les éléments de fermeture 18,19 à la sortie de la poche primaire 2
- ouvrir le clamp 45 pour permettre la communication fluidique entre la poche primaire 2 et la poche satellite de recueil du troisième composant sanguin 5,
- déplacer la plaque de pressage du réceptacle selon une première direction de sorte à compresser la poche primaire 2 et à chasser l'air de la poche primaire vers l'unité de filtration 6,
- à la détection par le détecteur 51 de l'interface air/plasma, fermer le clamp 45,
- ouvrir le clamp 44 pour permettre l'écoulement fluidique entre la poche primaire 2 et la poche filtrante 4,
- déplacer la plaque de contrainte 42 selon la première direction de sorte à contraindre le compartiment de stockage 10 du deuxième composant sanguin selon un premier volume,
- déplacer la plaque de pressage 24 du réceptacle 20 selon la première direction de sorte à compresser la poche primaire 2 et à transférer le premier composant sanguin dans la poche satellite 3 de recueil du premier composant sanguin en passant par le compartiment de stockage 10 et le compartiment de filtration 8 de la poche de stockage 7,
- à la détection par le détecteur 51 du deuxième composant sanguin, fermer le clamp 46 pour fermer le passage 11 entre le compartiment de stockage 10 de la poche filtrante 7 et le compartiment de filtration 8,
- déplacer la plaque de contrainte 42 selon la première direction de sorte à contraindre le compartiment de stockage 10 de la poche filtrante 7 selon un deuxième volume,
- déplacer la plaque de pressage 24 du réceptacle 20 selon la première direction de sorte à compresser la poche primaire 2 et à transférer le deuxième composant sanguin dans le compartiment de stockage 10 du deuxième composant sanguin,
- à la détection par le détecteur de pression placé sur la plaque de contrainte 42 d'une certaine valeur de pression correspondant à un volume déterminé de deuxième composant sanguin, fermer le clamp 44 pour interrompre la communication entre la poche primaire 2 et la poche filtrante 7,
- déplacer la plaque de pressage 24 dans une deuxième direction opposée à la première direction de sorte à compresser la poche satellite 3 de recueil du premier composant sanguin et à chasser l'air de ladite poche satellite,
- à la détection par le détecteur 52 de l'interface air/plasma, fermer le clamp 47 pour interrompre la communication entre la poche filtrante 7 et la poche satellite 3 de recueil du premier composant,
- ouvrir le clamp 45 pour permettre la communication entre la poche primaire 1 et la poche satellite 5 de recueil du troisième composant sanguin,
- déplacer la plaque de pressage 24 dans la deuxième direction de sorte à compresser la poche satellite 5 contenant la solution additive et à transférer ladite solution additive dans la poche primaire 2.

Une fois ces commandes exécutées, l'opérateur retire le système à poches 1 du réceptacle 20. Dans un exemple particulier, le composant globulaire et la solution additive contenus dans la poche primaire 2 sont ensuite filtrés par gravité en suspendant la poche primaire 2 à un portique.

## Revendications

1. Réceptacle (20) destiné à recevoir un système à poches (1) pour le traitement du sang comprenant une poche primaire (2) en communication fluidique avec au moins une poche satellite (3,5), ledit réceptacle comprenant une cavité (21) présentant un fond (22) et une paroi latérale (23), ladite cavité étant divisée par une plaque de pressage (24) en un compartiment primaire (25) destiné à recevoir la poche primaire (2) et en un compartiment secondaire (26) destiné à recevoir au moins une poche satellite (3,5) respectivement, **caractérisé en ce que** ladite plaque de pressage (24) est agencée pour être mobile à l'intérieur de ladite cavité (21) dans une première direction vers ladite paroi latérale (23) de sorte à pouvoir compresser la poche primaire (2).

2. Réceptacle selon la revendication 1, **caractérisé en ce que** la plaque de pressage (24) est mobile à l'intérieur de la cavité (21) dans une deuxième direction, opposée à la première direction, vers la paroi latérale (23) de sorte à pouvoir compresser ladite au moins une poche satellite (3,5).

3. Réceptacle selon la revendication 1 ou 2, **caractérisé en ce que** la plaque de pressage (24) comprend un logement pour une unité de filtration (6).

4. Réceptacle selon l'une quelconque des revendications 1 à 3, **caractérisé en ce que** la paroi latérale (23) est pourvue sur sa surface extérieure d'un moyen d'association d'une poche satellite (4,7).

5. Réceptacle selon l'une quelconque des revendications 1 à 4, **caractérisé en ce que** la plaque de pressage (24) est amovible.

6. Réceptacle selon l'une quelconque des revendications 1 à 5, **caractérisé en ce qu'**il est adapté pour être disposé dans un godet d'une centrifugeuse.

7. Appareil pour extraire au moins un composant sanguin contenu dans une poche primaire (2) d'un système à poches (1), ledit système à poches comprenant en outre au moins une poche satellite (3,5) de recueil dudit composant sanguin, ledit appareil comprenant :
- un réceptacle (20) selon l'une quelconque des revendications 1 à 6,
- un moyen de réception (38) dudit réceptacle, et
- un dispositif de déplacement (39) de la plaque de pressage (24) dans ledit réceptacle.

8. Appareil selon la revendication 7, **caractérisé en ce qu'**il comprend une plaque de contrainte (42) mobile pour contraindre une poche satellite (4) disposée sur la surface extérieure du réceptacle (20) et un dispositif de déplacement de ladite plaque de contrainte.

9. Appareil selon la revendication 7 ou 8, **caractérisé en ce qu'**il comprend en outre un dispositif de contrôle destiné à contrôler l'écoulement des fluides dans le système à poches (1).

10. Appareil selon la revendication 9, **caractérisé en ce que** le dispositif de contrôle comprend un ensemble de clamps (43,44,45) pour les tubulures associant entre elles la poche primaire (2) et les poches satellites (3,4,5).

11. Appareil selon l'une quelconque des revendications 7 à 10, **caractérisé en ce qu'**il comprend en outre un dispositif de détection afin de détecter l'interface entre les différents composants sanguins et/ou le passage d'un composant dans les tubulures du système à poches (1).

12. Appareil selon la revendication 11, **caractérisé en ce que** le dispositif de détection comprend un ensemble de détecteurs optiques (46,48) placés au niveau de la poche primaire (2) et/ou des tubulures.

13. Appareil selon l'une quelconque des revendications 7 à 12, **caractérisé en ce qu'**il comprend en outre un système de pilotage apte à commander les dispositifs de déplacement et de contrôle.

14. Procédé pour extraire au moins un premier composant sanguin contenu dans une poche primaire (2) d'un système à poches (1) pour le traitement du sang, ladite poche primaire (2) étant en communication fluidique avec au moins une poche satellite (3) de recueil du premier composant sanguin, ledit procédé étant réalisé à l'aide d'un appareil selon l'une quelconque des revendications 7 à 13, ledit procédé comprenant les étapes de :
- disposer le système à poches (1) dans un réceptacle (20) selon l'une des revendications 1 à 6,
- disposer le réceptacle (20) contenant le système à poches (1) sur le moyen de réception (38) dudit appareil;
- mettre en fonction l'appareil pour extraire ledit au moins un premier composant sanguin de la poche primaire (2).

15. Procédé selon la revendication 14, **caractérisé en ce que** la mise en fonction de l'appareil comprend l'action de déplacer la plaque de pressage (24) du réceptacle (20) pour compresser la poche primaire (2) du système à poches (1) de sorte à extraire le premier composant de la poche primaire (2) et à le transférer dans la poche satellite (3).

16. Procédé selon la revendication 14 pour extraire en outre au moins un deuxième composant sanguin contenu dans la poche primaire (2), ledit système à poches (1) comprenant en outre une poche satellite (4) de recueil du deuxième composant sanguin disposée sur le chemin d'écoulement défini entre la poche primaire (2) et la poche satellite (3) de recueil du premier composant sanguin, ledit appareil comprenant une plaque de contrainte (42) mobile pour contraindre la poche satellite (4) de recueil du deuxième composant sanguin disposée sur la surface extérieure du réceptacle (20), **caractérisé en ce que** la mise en fonction de l'appareil comprend les actions de:
- déplacer la plaque de contrainte (42) pour contraindre la poche satellite (4) de recueil du deuxième composant sanguin selon un premier volume,
- déplacer la plaque de pressage (24) pour compresser la poche primaire (2) de sorte à extraire le premier composant sanguin de la poche primaire (2) et le transférer au moins en partie dans la poche satellite (3) de recueil du premier composant sanguin en passant par la poche satellite (4) de recueil du deuxième composant sanguin contrainte selon le premier volume,
- après l'extraction du premier composant sanguin, déplacer la plaque de contrainte (42) pour contraindre la poche satellite (4) de recueil du deuxième composant sanguin selon un deuxième volume,
- déplacer la plaque de pressage (24) pour poursuivre la compression de la poche primaire (2) de sorte à extraire le deuxième composant sanguin de la poche primaire (2) et le transférer au moins en partie dans la poche satellite (4) de recueil du deuxième composant sanguin contrainte selon le deuxième volume.

17. Procédé selon la revendication 14 pour extraire en outre au moins un deuxième composant sanguin contenu dans la poche primaire (2), ledit système à poches (2) comprenant en outre une poche filtrante (7) disposée sur le chemin d'écoulement défini entre la poche primaire (2) et la poche satellite (3) de recueil du premier composant sanguin, la poche filtrante (7) comprenant un compartiment de filtration (8) disposant d'un milieu filtrant et un compartiment de stockage (10) du deuxième composant sanguin, lesdits compartiments (8,10) communiquant entre eux par un passage (11) pouvant être fermé, ledit appareil comprenant en outre un dispositif de contrôle destiné à contrôler l'écoulement des fluides dans le système à poches (1), **caractérisé en ce que** la mise en fonction de l'appareil comprend les actions de :
- déplacer la plaque de pressage (24) pour compresser la poche primaire (2) de sorte à extraire le premier composant sanguin de la poche primaire (2) et à le transférer au moins en partie dans la poche satellite (3) de recueil du premier composant sanguin en passant par ladite poche filtrante (7),
- poursuivre le déplacement de la plaque de pressage (24) pour poursuivre la compression de la poche primaire (2) de sorte à extraire le deuxième composant sanguin de la poche primaire (2) et à le transférer au moins en partie dans le compartiment de stockage (10) de ladite poche filtrante (7), et
- actionner le dispositif de contrôle pour fermer le passage (11) entre le compartiment de stockage (10) et le compartiment de filtration (8).

## Patentansprüche

1. Behälter (20) der dazu bestimmt ist, ein Beutelsystem (1) zur Behandlung von Blut aufzunehmen, einen Primärbeutel (2) in Fluidverbindung mit mindesten einem Satellitenbeutel (3, 5) umfassend, wobei der Behälter einen Hohlraum (21) umfasst, der einen Boden (22) und eine Seitenwand (23) aufweist, wobei der Hohlraum durch eine Pressplatte (24) in ein Primärfach (25), das dazu bestimmt ist, den Primärbeutel (2) aufzunehmen, und in ein Sekundärfach (26), das dazu bestimmt ist, jeweils mindestens einen Satellitenbeutel (3, 5) aufzunehmen, unterteilt wird, **dadurch gekennzeichnet, dass** die Pressplatte (24) angeordnet ist, um im Inneren des Hohlraumes (21) in einer ersten Richtung zu der Seitenwand (23) beweglich zu sein, um den Primärbeutel (2) zusammenpressen zu können.

2. Behälter nach Anspruch 1, **dadurch gekennzeichnet, dass** die Pressplatte (24) im Inneren des Hohlraumes (21) in einer zweiten Richtung, entgegengesetzt zur ersten Richtung, zu der Seitenwand (23) beweglich ist, um den mindestens einen Satellitenbeutel (3, 5) zusammenpressen zu können.

3. Behälter nach Anspruch 1 oder 2, **dadurch gekennzeichnet, dass** die Pressplatte (24) eine Aufnahme für eine Filtereinheit (6) umfasst.

4. Behälter nach einem der Ansprüche 1 bis 3, **dadurch gekennzeichnet, dass** die Seitenwand (23) an ihrer Außenfläche mit einem Mittel zum Zuweisen eines Satellitenbeutels (4, 7) versehen ist.

5. Behälter nach einem der Ansprüche 1 bis 4, **dadurch gekennzeichnet, dass** die Pressplatte (24) abnehmbar ist.

6. Behälter nach einem der Ansprüche 1 bis 5, **dadurch gekennzeichnet, dass** er angepasst ist, um in einem Napf einer Zentrifuge angeordnet zu werden.

7. Einrichtung zum Extrahieren mindestens eines Blutbestandteils, der in einem Primärbeutel (2) eines Beutelsystems (1) enthalten ist, wobei das Beutelsystem weiter mindestens einen Satellitenbeutel (3, 5) zum Sammeln des Blutbestandteils umfasst, wobei die Einrichtung umfass:
- einen Behälter (20) nach einem der Ansprüche 1 bis 6,
- ein Aufnahmemittel (38) für den Behälter, und
- eine Verschiebevorrichtung (39) der Pressplatte (24) in dem Behälter.

8. Einrichtung nach Anspruch 7, **dadurch gekennzeichnet, dass** sie eine bewegliche Spannplatte (42) zum Spannen eines Satellitenbeutels (4) umfasst, der an der Außenfläche des Behälters (20) angeordnet ist, und eine Verschiebevorrichtung der Spannplatte.

9. Einrichtung nach Anspruch 7 oder 8, **dadurch gekennzeichnet, dass** sie weiter eine Kontrollvorrichtung umfasst, die dazu bestimmt ist, die Strömung der Fluide in dem Beutelsystem (1) zu kontrollieren.

10. Einrichtung nach Anspruch 9, **dadurch gekennzeichnet, dass** die Kontrollvorrichtung eine Klemmeneinheit (43, 44, 45) für die Füllrohre umfasst, die den Primärbeutel (2) und die Satellitenbeutel (3, 4, 5) zueinander zuweisen.

11. Einrichtung nach einem der Ansprüche 7 bis 10, **dadurch gekennzeichnet, dass** sie weiter eine Detektionsvorrichtung umfasst, um die Schnittstelle zwischen den verschiedenen Blutbestandteilen und/oder den Durchgang eines Bestandteils in den Füllrohren des Beutelsystems (1) zu detektieren.

12. Einrichtung nach Anspruch 11, **dadurch gekennzeichnet, dass** die Detektionsvorrichtung eine Einheit an optischen Detektoren (46, 48) umfasst, die im Bereich des Primärbeutels (2) und/oder der Füllrohre platziert sind.

13. Einrichtung nach einem der Ansprüche 7 bis 12, **dadurch gekennzeichnet, dass** sie weiter ein Bediensystem umfasst, das imstande ist, die Verschiebe- und Kontrollvorrichtungen zu steuern.

14. Verfahren zum Extrahieren mindestens eines ersten Blutbestandteils, der in einem Primärbeutel (2) eines Beutelsystems (1) zur Behandlung des Bluts enthalten ist, wobei der Primärbeutel (2) in Fluidverbindung mit mindestens einem Satellitenbeutel (3) zum Sammeln des ersten Blutbestandteils ist, wobei das Verfahren mithilfe einer Einrichtung nach einem der Ansprüche 7 bis 13 umgesetzt wird, wobei das Verfahren die folgenden Schritte umfasst:
- Anordnen des Beutelsystems (1) in einem Behälter (20) nach einem der Ansprüche 1 bis 6,
- Anordnen des Behälters (20), der das Beutelsystem (1) enthält, auf dem Aufnahmemittel (38) der Einrichtung;
- Inbetriebnahme der Einrichtung zum Extrahieren des mindestens einen ersten Blutbestandteils aus dem Primärbeutel (2).

15. Verfahren nach Anspruch 14, **dadurch gekennzeichnet, dass** die Inbetriebnahme der Einrichtung die Tätigkeit des Verschiebens der Pressplatte (24) des Behälters (20) umfasst, um den Primärbeutel (2) des Beutelsystems (1) zusammenzupressen, um den ersten Bestandteil aus dem Primärbeutel (2) zu extrahieren und ihn in den Satellitenbeutel (3) zu transferieren.

16. Verfahren nach Anspruch 14, zum weiteren Extrahieren mindestens eines zweiten Blutbestandteils, der in dem Primärbeutel (2) enthalten ist, wobei das Beutelsystem (1) weiter einen Satellitenbeutel (4) zum Sammeln des zweiten Blutbestandteils umfasst, der auf dem Strömungsweg angeordnet ist, der zwischen dem Primärbeutel (2) und dem Satellitenbeutel (3) zum Sammeln des ersten Blutbestandteils definiert ist, wobei die Einrichtung eine bewegliche Spannplatte (42) zum Spannen des Satellitenbeutels (4) zum Sammeln des zweiten Blutbestandteils umfasst, der an der Außenfläche des Behälters (20) angeordnet ist, **dadurch gekennzeichnet, dass** die Inbetriebnahme der Einrichtung die folgenden Tätigkeiten umfasst:
- Verschieben der Spannplatte (42) zum Spannen des Satellitenbeutels (4) zum Sammeln des zweiten Blutbestandteils entsprechend einem ersten Volumen,
- Verschieben der Pressplatte (24) zum Zusammenpressen des Primärbeutels (2), um den ersten Blutbestandteil aus dem Primärbeutel (2) zu extrahieren und ihn mindestens teilweise über den Satellitenbeutel (4) zum Sammeln des zweiten Blutbestandteils, der entsprechend dem ersten Volumen gespannt ist, in den Satellitenbeutel (3) zum Sammeln des ersten Blutbestandteils zu transferieren,
- nach dem Extrahieren des ersten Blutbestandteils, Verschieben der Spannplatte (42) zum Spannen des Satellitenbeutels (4) zum Sammeln des zweiten Blutbestandteils entsprechend einem zweiten Volumen,
- Verschieben der Pressplatte (24) zum Fortsetzen des Zusammenpressens des Primärbeutels (2), um den zweiten Blutbestandteil aus dem Primärbeutel (2) zu extrahieren, und ihn mindestens teilweise in den Satellitenbeutel (4) zum Sammeln des zweiten Blutbestandteils zu transferieren, der entsprechend dem zweiten Volumen gespannt ist.

17. Verfahren nach Anspruch 14, zum weiteren Extrahieren mindestens eines zweiten Blutbestandteils, der in dem Primärbeutel (2) enthalten ist, wobei das Beutelsystem (2) weiter einen Filterbeutel (7) umfasst, der auf dem Strömungsweg angeordnet ist, der zwischen dem Primärbeutel (2) und dem Satellitenbeutel (3) zum Sammeln des ersten Blutbestandteils definiert ist, wobei der Filterbeutel (7) ein Filterfach (8) umfasst, das über ein Filtermedium und ein Lagerfach (10) für den zweiten Blutbestandteil verfügt, wobei die Fächer (8, 10) miteinander durch einen Durchgang (11) verbunden sind, der geschlossen werden kann, wobei die Einrichtung weiter eine Kontrollvorrichtung umfasst, die dazu bestimmt ist, die Strömung der Fluide in dem Beutelsystem (1) zu kontrollieren, **dadurch gekennzeichnet, dass** die Inbetriebnahme der Einrichtung die folgenden Tätigkeiten umfasst:
- Verschieben der Pressplatte (24) zum Zusammenpressen des Primärbeutels (2), um den ersten Blutbestandteil aus dem Primärbeutel (2) zu extrahieren und ihn mindestens teilweise über den Filterbeutel (7) in den Satellitenbeutel (3) zum Sammeln des ersten Blutbestandteils zu transferieren,
- Fortsetzen der Verschiebung der Pressplatte (24) zum Fortsetzen des Zusammenpressens des Primärbeutels (2) um den zweiten Blutbestandteil aus dem Primärbeutel (2) zu extrahieren, und ihn mindestens teilweise in das Lagerfach (10) des Filterbeutels (7) zu transferieren,
und
- Betätigen der Kontrollvorrichtung zum Schließen des Durchgangs (11) zwischen dem Lagerfach (10) und dem Filterfach (8).

## Claims

1. A receptacle (20) intended to receive a system (1) of bags for the treatment of blood comprising a primary bag (2) in fluid communication with at least one satellite bag (3, 5), said receptacle comprising a cavity (21) having a bottom (22) and a side wall (23), said cavity being divided by a pressing plate (24) into a primary compartment (25) intended to receive the primary bag (2) and into a secondary compartment (26) intended to receive at least one satellite bag (3, 5) respectively, **characterised in that** said pressing plate (24) is arranged to be movable inside said cavity (21) in a first direction towards said side wall (23) so as to be able to compress the primary bag (2).

2. The receptacle according to claim 1, **characterised in that** the pressing plate (24) is movable inside the cavity (21) in a second direction, opposite to the first direction, towards the side wall (23) so as to be able to compress said at least one satellite bag (3, 5).

3. The receptacle according to claim 1 or 2, **characterised in that** the pressing plate (24) comprises a housing for a filtration unit (6).

4. The receptacle according to any one of claims 1 to 3, **characterised in that** the side wall (23) is provided on its outer surface with a means for associating a satellite bag (4, 7).

5. The receptacle according to any one of claims 1 to 4, **characterised in that** the pressing plate (24) is removable.

6. The receptacle according to any one of claims 1 to 5, **characterised in that** it is adapted to be disposed in a bucket of a centrifuge.

7. An apparatus for extracting at least one blood component contained in a primary bag (2) of a system (1) of bags, said system of bags further comprising at least one satellite bag (3, 5) for collecting said blood component, said apparatus comprising:
- a receptacle (20) according to any one of claims 1 to 6,
- a means for receiving (38) said receptacle, and
- a device (39) for displacing the pressing plate (24) in said receptacle.

8. The apparatus according to claim 7, **characterised in that** it comprises a movable constraint plate (42) to constrain a satellite bag (4) disposed on the outer surface of the receptacle (20) and a device for displacing said constraint plate.

9. The apparatus according to claim 7 or 8, **characterised in that** it further comprises a control device intended to control the flow of fluids in the system (1) of bags.

10. The apparatus according to claim 9, **characterised in that** the control device comprises a set of clamps (43, 44, 45) for the tubing associating the primary bag (2) and the satellite bags (3, 4, 5) with each other.

11. The apparatus according to any one of claims 7 to 10, **characterised in that** it further comprises a detection device in order to detect the interface between the various blood components and/or the passage of a component in the tubing of the system (1) of bags.

12. The apparatus according to claim 11, **characterised in that** the detection device comprises a set of optical detectors (46, 48) placed at the primary bag (2) and/or the tubing.

13. The apparatus according to any one of claims 7 to 12, **characterised in that** it further comprises a piloting system capable of commanding the displacement and control devices.

14. A method for extracting at least a first blood component contained in a primary bag (2) of a system (1) of bags for the treatment of blood, said primary bag (2) being in fluid communication with at least one satellite bag (3) for collecting the first blood component, said method being carried out using an apparatus according to any one of claims 7 to 13, said method comprising the steps of:
- disposing the system (1) of bags in a receptacle (20) according to one of claims 1 to 6,
- disposing the receptacle (20) containing the system (1) of bags on the means (38) for receiving said apparatus;
- activating the apparatus to extract said at least a first blood component from the primary bag (2).

15. The method according to claim 14, **characterised in that** the activation of the apparatus comprises the action of displacing the pressing plate (24) of the receptacle (20) to compress the primary bag (2) of the system (1) of bags so as to extract the first component from the primary bag (2) and transfer it into the satellite bag (3).

16. The method according to claim 14 for further extracting at least a second blood component contained in the primary bag (2), said system (1) of bags further comprising a satellite bag (4) for collecting the second blood component disposed on the flow path defined between the primary bag (2) and the satellite bag (3) for collecting the first blood component, said apparatus comprising a movable constraint plate (42) to constrain the satellite bag (4) for collecting the second blood component disposed on the outer surface of the receptacle (20), **characterised in that** the activation of the apparatus comprises the actions of:
- displacing the pressing plate (42) to constrain the satellite bag (4) for collecting the second blood component according to a first volume,
- displacing the pressing plate (24) to compress the primary bag (2) so as to extract the first blood component from the primary bag (2) and at least partly transfer it into the satellite bag (3) for collecting the first blood component passing through the satellite bag (4) for collecting the second blood component constrained according to the first volume,
- after extracting the first blood component, displacing the constraint plate (42) to constrain the satellite bag (4) for collecting the second blood component according to a second volume,
- displacing the pressing plate (24) to continue the compression of the primary bag (2) so as to extract the second blood component from the primary bag (2) and at least partly transfer it into the satellite bag (4) for collecting the second blood component constrained according to the second volume.

17. The method according to claim 14 for further extracting at least a second blood component contained in the primary bag (2), said system of bags (2) further comprising a filter bag (7) disposed on the flow path defined between the primary bag (2) and the satellite bag (3) for collecting the first blood component, the filter bag (7) comprising a filtration compartment (8) having a filtering medium and a compartment (10) for storing the second blood component, said compartments (8,10) communicating with each other by a passage (11) which can be closed, said apparatus further comprising a control device intended to control the flow of fluids in the system (1) of bags, **characterised in that** the activation of the apparatus comprises the actions of:
- displacing the pressing plate (24) to compress the primary bag (2) so as to extract the first blood component from the primary bag (2) and to at least partly transfer it into the satellite bag (3) for collecting the first blood component by passing through said filter bag (7),
- continuing the displacement of the pressing plate (24) to continue compressing the primary bag (2) so as to extract the second blood component from the primary bag (2) and to at least partly transfer it into the storage compartment (10) of said filter bag (7), and
- actuating the control device to close the passage (11) between the storage compartment (10) and the filtration compartment (8).
